# EUROPÄISCHE PATENTSCHRIFT

(11) **EP 0 889 360 B1**
(45) Veröffentlichungstag und Bekanntmachung des Hinweises auf die Patenterteilung: **30.01.2002**
(21) Anmeldenummer: 98111153.7
(22) Anmeldetag: 17.06.1998
(51) Int. Cl.: G03F 7/029

(54) **Reaktionsharzmischungen und deren Verwendung**
Reactive resin mixtures and their use
Mélanges de résines réactives et leur utilisation

(30) Priorität: 30.06.1997 DE 19727822
(43) Veröffentlichungstag der Anmeldung: 07.01.1999
(73) Patentinhaber: SIEMENS AKTIENGESELLSCHAFT, 80333 München (DE)
(72) Erfinder: Schön, Lothar, Dipl.-Ing., 91077 Neunkirchen (DE); Rogler, Wolfgang, Dr., 91096 Möhrendorf (DE); Muhrer, Volker, Dipl.-Ing. (FH), 90768 Fürth (DE); Fedtke, Manfred, Prof. Dr., 06217 Merseburg (DE); Palinsky, Andreas, Dipl.-Ing., 30823 Garbsen (DE)

(56) Entgegenhaltungen:
- EP-A- 0 413 087
- EP-A- 0 738 928
- DE-A- 4 035 425
- US-A- 4 442 197
- US-A- 5 141 969

## Beschreibung

Die Erfindung betrifft härtbare Reaktionsharzmischungen, insbesondere zur Herstellung dreidimensionaler Kunststoffmodelle mittels Stereolithographie, sowie deren Verwendung.

Die Stereolithographie ist ein Verfahren beim sogenannten Rapid Prototyping Engineering (RPE), mit dem rasch beliebig komplizierte Kunststoffmodelle mit Hilfe von 3D-CAD-Konstruktionsdaten hergestellt werden können. Bei diesem Verfahren wird eine dünne Schicht eines flüssigen Reaktionsharzes mit einem rechnergesteuerten Laserstrahl belichtet und dabei stellenweise ausgehärtet. Das auf einer Plattform befindliche (teil-)gehärtete Gebilde wird dann um eine Schichtdicke in das in einem Behälter befindliche Reaktionsharz abgesenkt, und nach dem Beschichten (mit frischem Reaktionsharz) wird diese neue Schicht wieder teilweise mit dem Laser bestrahlt und gehärtet. Auf diese Weise entsteht Schicht für Schicht das dreidimensionale Modell.

Die bei diesem Prozeß verwendeten Reaktionsharzmischungen, sogenannte Photopolymere, müssen eine Reihe von teilweise gegenläufigen Anforderungen erfüllen:
- Hohe Reaktivität, die eine Verfestigung der gewählten Schichtdicke bei minimaler Expositionsenergie, d.h. eine hohe Verfahrgeschwindigkeit des Lasers ermöglicht und damit ausschlaggebend ist für kurze und deshalb ökonomische Herstellungszeiten.
- Hohe Grünteilfestigkeit, d.h. ausreichende mechanische Festigkeit des im Photopolymerbad entstehenden, nur teilgehärteten Bauteils. Dies ist für einen maßgenauen Herstellungsprozeß und ein zerstörungsfreies Handling erforderlich, bis das Bauteil nachgehärtet ist.
- Minimaler Schwund während der Härtung, was minimalen Curl während des Herstellungsprozesses und damit optimale Maßgenauigkeit bedeutet. Ausschlaggebend für geringen Curl ist der lineare Schwund in horizontaler Richtung, da aufgrund des schichtweisen Herstellungsprozesses immer eine teilgehärtete Schicht mit einer sich neu bildenden, ca. 100 µm dünnen Schicht verknüpft wird. Durch den Reaktionsschwund der neu generierten Schicht wird eine Spannung auf die darunter liegenden teilgehärteten Schichten ausgeübt, die zu Verzügen bzw. zu den bekannten Curl-Erscheinungen führt.
- Gute mechanische Eigenschaften der ausgehärteten Teile, die Funktionsprüfungen an den Bauteilen sowie die in der RPE-Technologie üblichen Folgeprozesse ermöglichen.
- Niedrige Viskosität von < 1500 mPa·s bei 25°C, was eine Voraussetzung für ein optimales Recoating ist, d.h. für kurze Wartezeiten bis zur Planarisierung der Photopolymerschichten nach dem Schichterzeugungsprozeß.

Aufgrund dieses komplexen Anforderungsspektums an Photopolymere für die Sterolithographie sind die meisten bekannten Harzmischungen aus mehreren Komponenten zusammengesetzt. Die derzeit reaktivsten Photopolymere für die Stereolithographie sind Mischungen aus acrylatfunktionellen Verbindungen, die im Rahmen einer radikalischen Polymerisation reagieren. Ein Nachteil dieser chemischen Basis ist jedoch ein relativ hoher Reaktionsvolumenschwund, der zu Maßabweichungen und Verformungen führt. Mit Acrylaten ist deshalb das ausgewogene Niveau mechanischer Eigenschaften technischer Thermoplaste im Hinblick auf hohe Festigkeit bei hoher Reißdehnung nicht erreichbar; es werden vielmehr im allgemeinen relativ spröde Formstoffe erhalten. Außerdem sind niedermolekulare Acrylate, wie sie zur Absenkung der Viskosität eingesetzt werden, aus arbeitshygienischen Gesichtspunkten bedenklich.

Eine deutliche Verbesserung im Hinblick auf mechanische Eigenschaften und Schwundverhalten stellen Epoxidharze dar. Diese können im Rahmen einer UV-initiierten kationischen Polymerisation gehärtet werden. Da die Härtungsgeschwindigkeit bei der kationischen Polymerisation von Epoxidharzen - verglichen mit der radikalischen Polymerisation von Acrylaten - geringer ist und die Härtung eine höhere UV-Dosis erfordert, werden üblicherweise Mischsysteme aus einer schnell härtenden Verbindungsklasse und Epoxidharzen eingesetzt. So sind aus WO 92/20014 kationisch härtbare Mischungen aus Vinylethern und Epoxidharzen und aus EP 0 605 361 A2 Mischungen aus Acrylaten und Epoxidharzen für die Stereolithographie bekannt.

Zur Kompensation der verminderten Reaktionsgeschwindigkeit kationisch härtbarer Photopolymerer ist es wünschenswert, möglichst leistungsstarke UV-Laser einzusetzen. Diese stehen beispielsweise mit Argon-Ionen-Lasern (mit Wellenlängen von 351 und 364 nm) sowie mit frequenzverdreifachten Nd:YAG-Lasern (mit einer Wellenlänge von 351 nm) auch zur Verfügung, die zur Initiierung der kationischen Polymerisation üblicherweise eingesetzten Triarylsulfoniumsalze (mit nicht-nucleophilen Anionen, wie Hexafluoroantimonat und Hexafluorophosphat) absorbieren aber nur im Wellenlängenbereich bis etwa 340 nm in ausreichendem Maße. Längerwelliges Licht wird dagegen nicht mehr effektiv absorbiert und kann somit praktisch nicht zur Auslösung einer Härtungsreaktion genutzt werden.

Bei der Stereolithographie ist es oft wünschenswert, durch besondere Belichtungsstrategien bewußt unbelichtete Teilbereiche im Kunststoffmodell zu belassen oder den Umsatz an funktionellen Gruppen in den belichteten Bereichen während der Laserbelichtung relativ gering zu halten. Dies kann sinnvoll sein, um Schwundeffekte zu minimieren oder auch, um Belichtungszeit einzusparen und auf diese Weise die Modelle rationeller, d.h. in kürzerer Zeit herstellen zu können. In diesen Fällen ist es jedoch erforderlich, diese Bereiche in einem nachfolgenden Prozeß auszuhärten. Eine nachträgliche Belichtung des fertigen Modells mit UV-Licht ist aber nicht effektiv. Für eine rasche Laserhärtung der dünnen Schichten müssen nämlich hohe Gehalte an UV-absorbierenden Initiatoren verwendet werden, was am fertigen Teil auch zu einer Absorption des Lichts in den oberflächennahen Schichten führt; tiefer liegende Bereiche sind deshalb einer UV-Nachhärtung nicht zugänglich. Ein geringerer Initiatorgehalt bzw. eine geringere optische Dichte würde eine Härtung auch in tieferen Bereichen, als dies erwünscht ist, bewirken und damit eine verschlechterte Maßhaltigkeit der Teile verursachen. Eine thermische Nachhärtung unbelichteter Bereiche ist aber nicht möglich, weil Triarylsulfoniumsalze thermisch sehr stabil sind. Eine Erhöhung der Temperatur, gleichzeitig mit der UV-Bestrahlung oder im Anschluß daran, bewirkt zwar eine Beschleunigung der Härtungsreaktion und eine Vervollständigung des Reaktionsumsatzes, diese Effekte werden aber nur an den Stellen beobachtet, an denen beim vorhergehenden UV-Prozeß Kationen aus dem Initiator entstanden sind. Alle anderen Bereiche bleiben unverändert flüssig.

Ein Bedarf an lagerstabilen, kationisch härtbaren Reaktionsharzen besteht auch auf Anwendungsgebieten, bei denen dickere Schichten erzeugt werden müssen, und auch dann, wenn lichtstreuende oder -absorbierende Zusatzstoffe, wie Füllstoffe, Pigmente und Farbstoffe, in der Mischung enthalten sind. Dann wird nämlich das Licht in den oberflächennahen Schichtbereichen sehr stark absorbiert bzw. gestreut, so daß das in tiefere Schichten transmittierte Licht nicht genügt, um eine ausreichende Härtung zu bewirken.

Ferner ist eine UV-Härtung nicht möglich, wenn verfahrensbedingt Bereiche vorhanden sind, die einer direkten Bestrahlung nicht zugänglich sind. Bei der Verklebung von nicht-transparenten Fügeteilen sowie von elektronischen Bauelementen und Baugruppen wird zunächst der Kleber appliziert und dann das Bauteil aufgesetzt. Durch Bestrahlung mit UV-Licht können dabei nur die Randbereiche gehärtet werden, an denen der Kleber hervorquillt, unter dem Bauteil muß die Härtung dagegen durch einen zusätzlichen Prozeß, beispielsweise thermisch induziert erfolgen. Vergleichbar ist die Situation, wenn Bauelemente und Baugruppen zum Schutz vor Umgebungseinflüssen mit einem Schutzlack versehen werden. Aufgrund der Kapillarkräfte migriert der Lack nämlich unter die Bauteile und kann dort wiederum nicht durch Belichtung gehärtet werden.

Aus der US-PS 4 417 061 ist es bekannt, daß Phenacylsulfoniumsalze bei UV-Bestrahlung eine kationische Polymerisation auslösen können. Es ist aber auch bekannt, daß dies nur bis zu einer Wellenlänge von 330 nm effektiv möglich ist (siehe dazu: "Photopolymere", VEB Deutscher Verlag für Grundstoffindustrie, Leipzig 1988, Seite 105); im längerwelligen Bereich müssen Sensibilisatoren verwendet werden. So ist es beispielsweise aus der US-PS 4 442 197 bekannt, daß Dialkylphenacylsulfoniumsalze besonders mit aromatischen Kohlenwasserstoffen sensibilisiert werden können. Weiterhin ist bekannt, daß Dialkylphenacylverbindungen - zusammen mit Reduktionsmitteln - in der Hitze eine kationische Polymerisation auslösen können. Dialkylphenacylverbindungen der genannten Art sind jedoch in den technisch eingesetzten kationisch polymerisierbaren Reaktionsharzmischungen schlecht löslich und kristallisieren im Laufe der Zeit wieder aus. Damit sind diese Verbindungen in der Praxis dann nicht verwendbar, wenn die Mischungen über einen längeren Zeitraum hinweg stabil bleiben müssen.

Es ist ferner bekannt, daß elektronenschiebende Substituenten in para-Stellung die Photolyse von Dialkylphenacylsulfoniumsalzen durch eine Stabilisierung kationischer Zwischenprodukte beschleunigen ("Macromolecules", Vol. 16 (1983), Seiten 864 bis 870). Dies entspricht Befunden, nach denen die thermische Reaktivität von 4-Methoxybenzylthiolanium-hexafluoroantimonat im Vergleich mit der unsubstituierten Verbindung wesentlich höher ist ("Makromolekulare Chemie", Bd. 192 (1991), Seiten 655 bis 662); dabei wird nämlich bereits bei Temperaturen ab ca. 40°C eine effektive kationische Polymerisation von Styrol beobachtet. Bei derartigen Mischungen ist aber nicht zu erwarten, daß die Viskosität bei Raumtemperatur über Wochen hinweg konstant bleibt.

Aufgabe der Erfindung ist es, lagerstabile, UV- und thermisch nach einem kationischen Mechanismus härtbare Reaktionsharzmischungen bereitzustellen, die mit UV-Licht im Wellenlängenbereich von etwa 350 bis 400 nm initiierbar sind, beispielsweise mit den Wellenlängen des Argon-Ionen-Lasers bei 351 und 364 nm, und auch thermisch ausgehärtet werden können, und zwar - durch eine nachträgliche Temperung - insbesondere in zuvor nicht UV-bestrahlten Volumenelementen.

Dies wird erfindungsgemäß durch Reaktionsharzmischungen folgender Zusammensetzung erreicht:
- ein kationisch härtbares Monomer und/oder Oligomer (Komponente A),
- ein Initiator folgender Struktur: 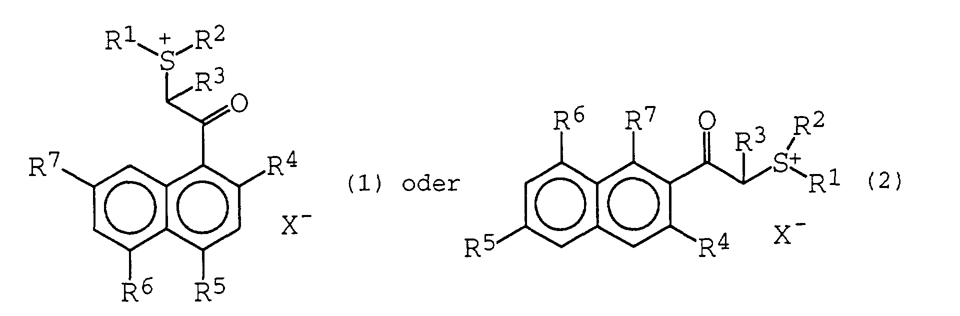 wobei folgendes gilt:
   R¹ und R² sind - unabhängig voneinander - Alkyl mit 1 bis 9 C-Atomen (linear oder verzweigt) oder Cycloalkyl mit 4 bis 9 C-Atomen oder bilden zusammen eine divalente aliphatische Gruppierung mit 4 bis 7 C-Atomen, d.h. zusammen mit dem S-Atom einen heterocyclischen Ring,
   R³ ist H oder Alkyl mit 1 bis 9 C-Atomen (linear oder verzweigt),
   R⁴, R⁵, R⁶ und R⁷ sind - unabhängig voneinander - H, Alkyl oder Alkoxy, jeweils mit 1 bis 9 C-Atomen (linear oder verzweigt), wobei mindestens einer der Reste R⁴ bis R⁷ Alkyl oder Alkoxy ist,
   X⁻ ist ein nicht-nucleophiles Anion, wie Hexafluoroantimonat (SbF₆⁻), -arsenat (AsF₆⁻) und -phosphat (PF₆⁻), Tetraphenylborat (B(C₆H₅)₄⁻), Tetra-(perfluorphenyl)-borat (B(C₆F₅)₄⁻) oder Trifluormethansulfonat (CF₃-SO₃⁻),
- und gegebenenfalls Füllstoff, Pigment und/oder Additiv.

Überraschenderweise hat sich gezeigt, daß kationisch härtbare Reaktionsharzmischungen die genannten Anforderungen erfüllen, wenn sie als Initiator eine Verbindung der vorstehenden Struktur enthalten. Derartige Reaktionsharzmischungen sind nämlich lagerstabil und sie können sowohl durch UV-Licht, insbesondere durch Laserstrahlung im Wellenlängenbereich von 350 bis 400 nm, als auch - in den nicht-belichteten Bereichen - allein durch Temperaturerhöhung gehärtet werden.

Als kationisch härtbare Monomere bzw. Oligomere (Komponente A) können allgemein Verbindungen verwendet werden, die zu einer kationischen Polymerisation befähigt sind, d.h. kationisch gehärtet werden können. Derartige Verbindungen sind beispielsweise aus der EP 0 126 712 B1 bekannt. Die Monomeren bzw. Oligomeren können auch im Gemisch vorliegen.

Die Komponente A ist vorzugsweise eine epoxy- oder vinyletherfunktionelle Verbindung. Geeignete epoxyfunktionelle Verbindungen sind insbesondere epoxidierte Terpene oder α-Alkene, cycloaliphatische Epoxidharze, Epoxyalkohole, Glycidylether und epoxyfunktionalisierte Silicone; als besonders vorteilhaft haben sich cycloaliphatische Epoxidharze erwiesen. Bevorzugt werden Verbindungen mit ≥ 2 Epoxidgruppen pro Molekül eingesetzt.

Als vinyletherfunktionelle Verbindungen kommen grundsätzlich alle vinyletherfunktionalisierten Hydroxylverbindungen in Frage. Geeignete Verbindungen sind insbesondere Cyclohexandimethyloldivinylether, Triethylenglykoldivinylether, Butandioldivinylether, Bis(4-vinyloxybutyl)-isophthalat, Bis-(4-vinyloxybutyl)-succinat, Bis(4-vinyloxymethylcyclohexylmethyl)-glutarat und Hydroxybutylmonovinylether oder vinyletherfunktionalisierte Hydroxypolyurethane mit aliphatischer oder aromatischer Grundstruktur. Bevorzugt sind Vinylether mit ≥ 2 Vinylethergruppen pro Molekül.

Die Reaktionsharzmischungen können zusätzlich noch hydroxylgruppenhaltige Verbindungen enthalten, d.h. polyfunktionelle Hydroxylverbindungen, die im Rahmen einer Kettenübertragungsreaktion am kationischen Reaktionsmechanismus beteiligt sind.

Derartige Verbindungen sind insbesondere Polyalkylenpolyole, Polyoxyalkylenpolyole und cycloaliphatische Hydroxylverbindungen; bevorzugt sind polyfunktionelle Hydroxylverbindungen mit ≥ zwei Hydroxylgruppen pro Molekül. Die Verwendung hydroxylgruppenhaltiger Verbindungen erweist sich als vorteilhaft, um die Reaktivität und den erreichbaren Reaktionsumsatz zu erhöhen und die entstehenden Formstoffe zu elastifizieren.

Die Initiatoren weisen die vorstehend angegebene Struktur (1) oder (2) auf. Bevorzugt werden dabei Verbindungen der Struktur (1), wobei R¹ und R² Methyl sind oder zusammen eine aliphatische Gruppierung mit vier Kohlenstoffatomen bilden und R⁴ und/oder R⁵ eine Methoxygruppe ist. Der Gehalt des Initiators in der Reaktionsharzmischung beträgt vorteilhaft etwa 0,01 bis 10 Masse-%, bezogen auf die Komponente A.

Die Initiatoren können zum Beispiel in der Weise hergestellt werden, daß die entsprechenden Thioether mit einem Alkylhalogenid umgesetzt werden. Aufgrund der höheren Polarisierbarkeit des Bromatoms und der daraus resultierenden leichteren Bromabspaltung stellen Alkylbromide - verglichen mit Alkylchloriden - die reaktiveren Ausgangsstoffe dar. Die Umsetzung, d.h. die Alkylierung, kann sowohl in aprotischen als auch in polar-protischen Lösemitteln durchgeführt werden. Polar-protische Lösemittel stabilisieren aufgrund ihrer guten Solvatationsfähigkeit die bei der Umsetzung gebildeten ionischen Produkte. Der Vorteil bei der Verwendung aprotischer Lösemittel besteht darin, daß die Produkte infolge der geringen Solvatation schwerlöslich sind, wodurch das chemische Gleichgewicht in gewünschter Weise verschoben wird. Im Anschluß an die Alkylierung erfolgt der Austausch des Halogenides gegen ein nicht-nucleophiles Anion.

Wird die Alkylierung in einem aprotischen Lösemittel durchgeführt, so werden beispielsweise 100 mmol des betreffenden Alkylhalogenides mit der äquimolaren Menge des Thioethers in 50 ml Aceton versetzt, und dann wird bei Raumtemperatur eine Stunde gerührt. Nach etwa 24 h wird der ausgefallene Niederschlag abgesaugt, mit kaltem Aceton gewaschen und im Vakuum getrocknet.

Zur Durchführung des Anionenaustausches wird das Sulfoniumhalogenid, je nach Löslichkeit, in möglichst wenig Methanol gelöst und mit der äquimolaren Menge Natriumhexafluoroantimonat (Na[SbF₆]), das unter leichtem Erwärmen in Methanol gelöst wird, versetzt. Der ausgefallene Niederschlag wird abfiltriert und in Methanol bzw. in einem Methanol/Aceton-Lösemittelgemisch mehrmals umkristallisiert, bis die Halogenidprobe mit AgNO₃ negativ ist.

Erfolgt die Umsetzung in einem polar-protischen Lösemittel, so werden beispielsweise je 50 mmol des Thioethers und des Alkylhalogenides in 100 ml Methanol gelöst, und dann wird 12 h bei Raumtemperatur gerührt. Anschließend werden der Reaktionsmischung 60 mmol Na[SbF₆], in 60 ml Methanol bei 40°C gelöst, zugesetzt. Nach weiteren 12 h wird der ausgefallene Niederschlag abfiltriert und wie vorstehend beschrieben umkristallisiert.

In den Reaktionsharzmischungen nach der Erfindung können - zusätzlich zur kationisch härtbaren Komponente - radikalisch härtbare Verbindungen enthalten sein. Dies sind insbesondere Verbindungen wie Acrylsäureester und Methacrylsäureester, die vorzugsweise bis zu einem Gehalt von ca. 30 Masse-%, bezogen auf die gesamte Harzbasis, vorhanden sind. In diesem Fall können die Reaktionsharzmischungen ferner etwa 0,1 bis 10 Masse-%, bezogen auf die radikalisch härtbare Verbindung, eines - an sich bekannten - unter UV-Bestrahlung Radikale bildenden Initiators aufweisen.

Zur Modifizierung der Verarbeitungs- und Formstoffeigenschaften können die Reaktionsharzmischungen Zusatzstoffe, wie mineralische und organische Füllstoffe, Farbstoffe, Pigmente, Stabilisatoren, Thixotropiermittel, Benetzungsmittel und Haftvermittler, enthalten.

Die Reaktionsharzmischungen nach der Erfindung können sowohl durch Bestrahlung mit UV-Licht als thermisch gehärtet werden. In lichtabgeschatteten Bereichen oder solchen Bereichen, die - verfahrensbedingt - durch UV-Belichtung nur teilweise ausgehärtet werden, kann die Härtung - gleichzeitig mit der UV-Bestrahlung oder in einem nachfolgenden Prozeß - durch eine Temperaturerhöhung erfolgen. Die Härtungstemperatur liegt im allgemeinen zwischen 80 und 200°C, vorzugsweise etwa bei 80 bis 150°C.

Zur UV-Belichtung können prinzipiell alle üblichen UV-Quellen eingesetzt werden, wie Xenon-, Wolfram-, Quecksilber- und Metallhalogenidstrahler; ferner ist der Einsatz von UV-Lasern möglich. Die Laserstrahlung kann mit Hilfe eines optischen Systems fokussiert sein; die UV-Emission kann sowohl kontinuierlich als auch gepulst erfolgen. Bevorzugt ist der Einsatz von UV-Licht im Wellenlängenbereich von 350 bis 400 nm. Es ist möglich, Schichten aus den Reaktionsharzmischungen vollflächig durch UV-Bestrahlung auszuhärten oder nur lokal begrenzte Bereiche. Die lokale Begrenzung der Härtung kann beispielsweise durch Belichtung über eine Maske erreicht werden. Eine weitere Möglichkeit besteht darin, diese Bereiche mit einem computergesteuerten Laserstrahl zu belichten.

Die kationisch härtbaren Reaktionsharzmischungen nach der Erfindung eignen sich zur Beschichtung oder Verklebung von Bauteilen, insbesondere von elektronischen Bauelementen und Baugruppen, und zwar vor allem dann, wenn verfahrensbedingt lichtabgeschattete Bereiche auftreten und/oder die Eindringtiefe des UV-Lichtes zu gering ist für eine vollständige Aushärtung. Dies ist beispielsweise dann der Fall, wenn nichttransparente Teile verklebt werden; durch UV-Belichtung der lichtzugänglichen Randbereiche kann eine Fixierung und durch einen thermischen Prozeß auch eine Härtung zwischen den Fügepartnern erreicht werden. Reaktionsharzmischungen, die zur Modifizierung der Formstoffeigenschaften lichtstreuende oder -absorbierende Zusätze, wie Füllstoffe, Farbstoffe, Pigmente und Stabilisatoren, enthalten, können durch UV-Bestrahlung oberflächlich oder teilweise gehärtet werden; eine vollständige Härtung ist dann wiederum durch einen thermischen Prozeß möglich.

Die Reaktionsharzmischungen können ferner zur Erzeugung von Strukturen dienen. Dazu wird durch ein geeignetes Verfahren eine Schicht aus der Reaktionsharzmischung hergestellt und diese Schicht durch eine Maske oder mittels eines Laserstrahls belichtet. Die unbelichteten Bereiche werden dann mit einem geeigneten Lösemittel herausgelöst.

Vorzugsweise werden die Reaktionsharzmischungen nach der Erfindung zur stereolithographischen Herstellung dreidimensionaler Gebilde eingesetzt, wobei anhand von 3D-CAD-Daten beliebig komplizierte Kunststoffmodelle realisiert werden können. Dazu wird (in einem Behälter) eine dünne Schicht der Reaktionsharzmischung mittels eines Lasers bildmäßig belichtet und dabei an den Stellen gehärtet, die der unteren Teilfläche des herzustellenden Modells entsprechen. Dabei entsteht eine erste Schicht der dreidimensionalen Struktur. Anschließend wird über dieser ersten Schicht eine weitere dünne Schicht der Reaktionsharzmischung ausgebildet und dann entsprechend belichtet, d.h. gehärtet. Dabei entsteht eine zweite Schicht der dreidimensionalen Struktur, die sich mit der ersten Schicht verbindet. Diese Verfahrensschritte werden so oft wiederholt, bis die dreidimensionale Struktur vollständig schichtweise aufgebaut ist. Zur Erhöhung der Produktivität ist es vorteilhaft, die einzelnen Teilschichten nicht durch eine hohe UV-Dosis vollständig auszuhärten, sondern beispielsweise lediglich die äußere Kontur und im Inneren ein Gitternetz auszuhärten oder die UV-Dosis über die Laserverfahrgeschwindigkeit so einzustellen, daß in der Schicht zwar eine Verfestigung erzielt, aber kein vollständiger Umsatz erreicht wird. In diesen Fällen kann das fertige Modell im Anschluß an den schichtweisen Aufbau und erforderlichenfalls nach einer Reinigung (nach Entnahme aus dem Behälter) durch Temperung und/oder UV-Belichtung auch im Inneren vollständig ausgehärtet werden.

Anhand von Ausführungsbeispielen soll die Erfindung noch näher erläutert werden.

Tabelle 1 gibt einen Überblick über die in den nachfolgenden Beispielen eingesetzten Initiatoren, welche folgende Struktur aufweisen:

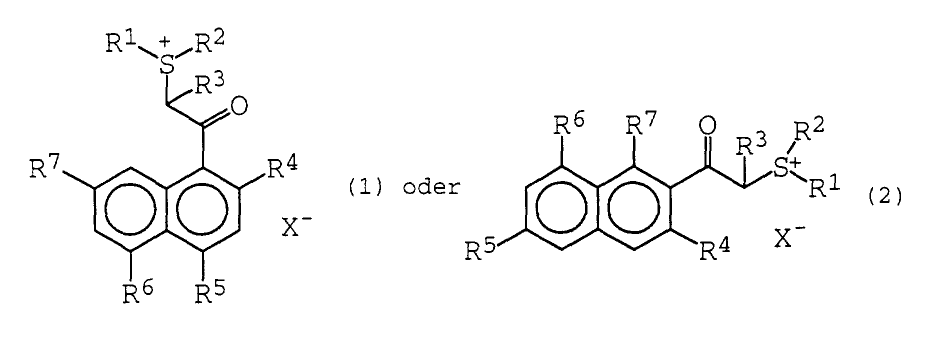

**Tabelle 1**

| Initiator | A | B | C | D | E | F |
|---|---|---|---|---|---|---|
| Struktur | 2 | 1 | 1 | 1 | 1 | 1 |
| R¹ | R¹ und R² bilden zusammen eine Tetramethylengruppierung -(CH₂)₄- | | | | | -CH₃ |
| R² | | | | | | -CH₃ |
| R³ | -H | -H | -H | -H | -H | -H |
| R⁴ | -H | -H | -H | -OCH₃ | -CH₃ | -H |
| R⁵ | -OCH₃ | -CH₃ | -OCH₃ | -H | -H | -OCH₃ |
| R⁶ | -H | -H | -H | -H | -H | -H |
| R⁷ | -H | -H | -H | -H | -H | -H |
| X⁻ | SbF₆⁻ | SbF₆⁻ | SbF₆⁻ | SbF₆⁻ | SbF₆⁻ | SbF₆⁻ |

| Elementaranalyse | | | | | | |
|---|---|---|---|---|---|---|
| C [%] (ber.) | 39,0 | 40,3 | 39,0 | 39,0 | 40,3 | 36,2 |
| C [%] (gef.) | 38,9 | 40,4 | 39,0 | 39,2 | 40,2 | 36,0 |
| H [%] (ber.) | 3,7 | 3,8 | 3,7 | 3,7 | 3,8 | 3,4 |
| H [%] (gef.) | 3,8 | 4,0 | 3,4 | 3,7 | 3,5 | 3,4 |
| S [%] (ber.) | 6,1 | 6,3 | 6,1 | 6,1 | 6,3 | 6,4 |
| S [%] (gef.) | 6,1 | 6,3 | 6,2 | 6,0 | 6,3 | 6,4 |

| "ber." = berechnet, "gef." = gefunden | | | | | | |
|---|---|---|---|---|---|---|
| Schmelzpunkt [°C] | >260 | 209 | 199-200 | 180-183 | 195 | 226-228 |

Die nachfolgenden Beispiele 1 bis 18 zeigen, daß die Reaktionsharzmischungen nach der Erfindung sowohl rein thermisch als auch durch UV-Bestrahlung gehärtet werden können. Die Zusammensetzung der Harzmischungen (in Masseteilen) ist in Tabelle 2 angegeben.

### Beispiele 1 bis 6

Zur Herstellung einer Harzbasis werden gleiche Masseteile Bisphenol-A-diglycidylether und Cyclohexandimethyloldivinylether unter Erwärmen auf ca. 50°C unter Rühren gelöst. Aus dem jeweiligen Initiator und 1,2-Propylencarbonat wird eine Lösung hergestellt, zu der die der Zusammensetzung nach Tabelle 2 entsprechende Menge an Harzbasis zugegeben wird.
Die erhaltene Reaktionsharzmischung wird dann bei Raumtemperatur unter Lichtausschluß gerührt und homogenisiert.

### Beispiele 7 bis 10

Zur Herstellung einer Harzbasis werden 95 Masseteile Bis-(epoxycyclohexyl-methyl)-adipat und 5 Masseteile Trimethylolpropan unter Erwärmen auf ca. 50°C unter Rühren gelöst. Aus dem jeweiligen Initiator und 1,2-Propylencarbonat wird eine Lösung hergestellt, zu der die der Zusammensetzung nach Tabelle 2 entsprechende Menge an Harzbasis zugegeben wird. Die erhaltene Reaktionsharzmischung wird dann bei Raumtemperatur unter Lichtausschluß gerührt und homogenisiert.

**Tabelle 2**

| Beispiel | 1 | 2 | 3 | 4 | 5 | 6 | 7 | 8 | 9 | 10 |
|---|---|---|---|---|---|---|---|---|---|---|
| Bisphenol-A-diglycidylether | 50 | 50 | 50 | 50 | 50 | 50 | - | - | - | - |
| Cyclohexandimethyloldivinylether | 50 | 50 | 50 | 50 | 50 | 50 | - | - | - | - |
| Bis(epoxycyclohexyl-methyl)-adipat | - | - | - | - | - | - | 95 | 95 | 95 | 95 |
| Trimethylolpropan | - | - | - | - | - | - | 5 | 5 | 5 | 5 |
| 1,2-Propylencarbonat | 1,6 | 1,6 | 1,6 | 1,6 | 2,0 | 1,6 | 1,6 | 1,6 | 1,6 | 1,6 |
| Initiator A | 0,4 | - | - | - | - | - | 0,4 | - | - | - |
| Initiator B | - | 0,4 | - | - | - | - | - | - | - | - |
| Initiator C | - | - | 0,4 | - | - | - | - | 0,4 | - | - |
| Initiator D | - | - | - | 0,4 | - | - | | - | 0,4 | - |
| Initiator E | - | - | - | - | 1,0 | - | - | - | - | - |
| Initiator F | - | - | - | - | - | 0,4 | - | - | - | 0,4 |

Die in Tabelle 3 wiedergegebenen Ergebnisse zeigen, daß die Reaktionsharzmischungen nach der Erfindung lagerstabil sind.

Außerdem ergibt sich aus DSC-Untersuchungen, daß diese Mischungen rein thermisch gehärtet werden können. Hierzu werden jeweils ca. 2 bis 3 mg der Reaktionsharzmischung in ein Probengefäß eingewogen, das dann verschlossen und von 25 bis 300°C mit einer Heizrate von 10 K/min aufgeheizt wird.
An der dabei erhaltenen Wärmestromkurve erfolgt eine Peakauswertung. Die Ergebnisse sind ebenfalls aus Tabelle 3 ersichtlich.

**Tabelle 3**

| Beispiel | 1 | 2 | 3 | 4 | 5 | 6 | 7 | 8 | 9 | 10 |
|---|---|---|---|---|---|---|---|---|---|---|
| Lagerstabilität [Monate] (Zeit bis zur Viskositätsverdoppelung) | > 6 | > 3 | > 6 | > 6 | > 3 | > 6 | > 3 | > 3 | > 3 | > 3 |

| Ergebnisse aus DSC-Untersuchungen (Heizrate 10 K/min) | | | | | | | | | | |
|---|---|---|---|---|---|---|---|---|---|---|
| Onset [°C] | 125 | 114 | 122 | 122 | 107 | 124 | 145 | 150 | 137 | 149 |
| Peakmaximum [°C] | 134 | 124 | 132 | 131 | 118 | 135 | 159 | 165 | 155 | 166 |
| Enthalpie [J/g] | 360 | 600 | 490 | 406 | 550 | 306 | 405 | 420 | 407 | 380 |

### Beispiele 11 bis 16

Photokalorimetrische Untersuchungen zeigen, daß die Reaktionsharzmischungen nach der Erfindung durch UV-Bestrahlung gehärtet werden können. Hierzu wird jeweils ca. 1 mg der Reaktionsharzmischung in ein Probenpfännchen eingewogen, und dann wird in einem Leistungsdifferenzkalorimeter während eines isothermen Meßlaufes bei 40°C mit monochromatischem UV-Licht der Wellenlänge 351 nm bestrahlt. An der dabei erhaltenen Wärmestromkurve erfolgt eine Peakauswertung. Die Ergebnisse sind aus Tabelle 4 ersichtlich.

**Tabelle 4**

| Beispiel | 11 | 12 | 13 | 14 | 15 | 16 |
|---|---|---|---|---|---|---|
| Mischung nach Beispiel | 2 | 3 | 5 | 6 | 8 | 10 |

| Ergebnisse aus Photo-DSC-Untersuchungen | | | | | | |
|---|---|---|---|---|---|---|
| Zeit bis zum Peakmaximum [s] | 8,0 | 4,3 | 9,2 | 6,3 | 6,3 | 5,8 |
| Peakhöhe [W/g] | 4,4 | 17,4 | 4,1 | 8,1 | 7,6 | 5,6 |
| Enthalpie [J/g] | 305 | 261 | 235 | 265 | 176 | 208 |

### Beispiel 17

Mittels der Reaktionsharzmischung nach Beispiel 8 werden Leiterplattenflachbaugruppen im Tauchverfahren mit einem Schutzlack versehen und nachfolgend beidseitig 5 min mit UVA-Licht (50 mW/cm²) bestrahlt. Alle dem Licht zugänglichen Bereiche sind dann klebfrei verfestigt, während die unter integrierten Schaltkreisen befindlichen Harzbereiche zu diesem Zeitpunkt noch flüssig sind. Anschließend werden die Leiterplattenflachbaugruppen eine Stunde bei 125°C getempert. Danach sind auch die dem UV-Licht nicht direkt zugänglichen Bereiche ausgehärtet.

### Beispiel 18

Rechteckige Bereiche der Harzoberfläche einer Reaktionsharzmischung nach Beispiel 3 werden mittels eines rechnergesteuerten Laserstrahls mit unterschiedlicher Energie belichtet. Dabei werden überlappende parallele Linien mit einem Abstand von 0,05 mm auf die Harzoberfläche "geschrieben". Von Bereich zu Bereich wird die Laserverfahrgeschwindigkeit und damit die auf die Oberfläche aufgebrachte Energie variiert. Im Anschluß an die Belichtung werden die gehärteten Bereiche entnommen und in 2-Propanol gereinigt, dann wird die Schichtdicke vermessen. Die Ergebnisse (Abhängigkeit der gehärteten Schichttiefe von der aufgebrachten Energie) sind in Tabelle 5 wiedergegeben. Laserleistung: 65 mW; Linienabstand: 0,05 mm; Laser-Verfahrgeschwindigkeit: variabel von 1 bis 7 m/s.

**Tabelle 5**

| | | | | | | | | |
|---|---|---|---|---|---|---|---|---|
| Laserverfahrgeschwindigkeit | m/s | 1 | 2 | 3 | 4 | 5 | 6 | 7 |
| Energie (berechnet aus Laserleistung, Linienabstand und Verfahrgeschwindigkeit) | mJ/cm² | 130 | 65 | 43,3 | 32,5 | 26 | 21,7 | 18,6 |
| gehärtete Schichtdicke | mm | 0,29 | 0,24 | 0,15 | 0,12 | 0,11 | 0,07 | 0,04 |

Das beschriebene Verfahren wird bei Photopolymeren für die Stereolithographie verwendet (Ermittlung der Arbeitskurve), um deren Reaktivität zu quantifizieren. Die Schichtdicke wird gegen die Energie im halblogarithmischen Maßstab aufgetragen; der Schnittpunkt mit der Abszisse liefert die kritische Energie ("critical exposure") Ec, die Steigung der üblicherweise erhaltenen Geraden wird als Eindringtiefe ("penetration depth") Dp bezeichnet. Für Beispiel 18 wird dabei ein Ec-Wert von 13,0 mJ/cm² und ein Dp-Wert von 0,14 mm ermittelt.

Beispiel 18 zeigt, daß die Reaktionsharzmischungen nach der Erfindung durch selektive Belichtung mit einem Argon-Ionen-Laser (Wellenlänge 351 und 364 nm) effektiv gehärtet werden können. Die erhaltenen Werte belegen, daß diese Reaktionsharzmischungen ausreichend reaktiv für die Verwendung in der Stereolithographie sind.

## Patentansprüche

1. Härtbare Reaktionsharzmischungen, insbesondere für die Stereolithographie, **gekennzeichnet durch** folgende Zusammensetzung:
- ein kationisch härtbares Monomer und/oder Oligomer (Komponente A),
- ein Initiator folgender Struktur: 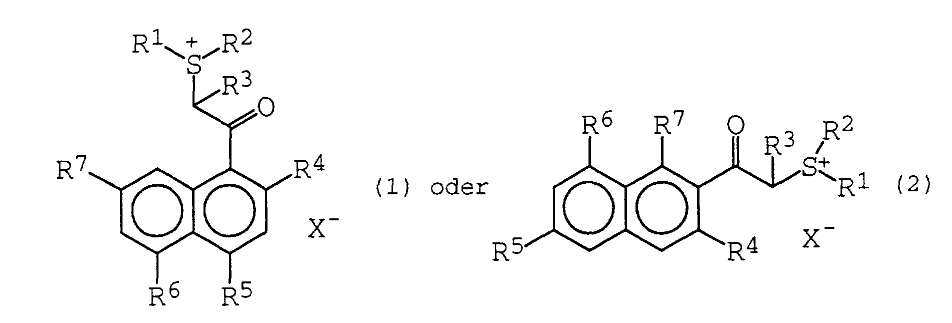 wobei folgendes gilt:
R¹ und R² sind Alkyl mit 1 bis 9 C-Atomen oder Cycloalkyl mit 4 bis 9 C-Atomen oder bilden zusammen eine divalente aliphatische Gruppierung mit 4 bis 7 C-Atomen,
R³ ist H oder Alkyl mit 1 bis 9 C-Atomen,
R⁴, R⁵, R⁶ und R⁷ sind H, Alkyl mit 1 bis 9 C-Atomen oder Alkoxy mit 1 bis 9 C-Atomen, wobei mindestens einer der Reste R⁴ bis R⁷ Alkyl oder Alkoxy ist,
X⁻ ist ein nicht-nucleophiles Anion, wie Hexafluoroantimonat, -arsenat und -phosphat, Tetraphenylborat, Tetra-(perfluorphenyl)-borat oder Trifluormethansulfonat,
- und gegebenenfalls Füllstoff, Pigment und/oder Additiv.

2. Reaktionsharzmischungen nach Anspruch 1, **dadurch gekennzeichnet, daß** der Gehalt an Initiator 0,01 bis 10 Masse-% beträgt, bezogen auf die Komponente A.

3. Reaktionsharzmischungen nach Anspruch 1 oder 2, d a**durch gekennzeichnet**, daß die Komponente A eine epoxyfunktionelle Verbindung ist, insbesondere eine Verbindung mit mindestens zwei Epoxidgruppen pro Molekül.

4. Reaktionsharzmischungen nach Anspruch 3, **dadurch gekennzeichnet, daß** die epoxyfunktionelle Verbindung ein epoxidiertes Terpen oder α-Alken, ein cycloaliphatisches Epoxid, ein Epoxyalkohol, ein Glycidylether oder ein epoxyfunktionalisiertes Silicon ist.

5. Reaktionsharzmischungen nach Anspruch 1 oder 2, d a**durch gekennzeichnet**, daß die Komponente A eine vinyletherfunktionelle Verbindung ist, insbesondere eine Verbindung mit mindestens zwei Vinylethergruppen pro Molekül.

6. Reaktionsharzmischungen nach einem oder mehreren der Ansprüche 1 bis 5, **dadurch gekennzeichnet, daß** sie zusätzlich eine polyfunktionelle Hydroxylverbindung enthalten.

7. Reaktionsharzmischungen nach einem oder mehreren der Ansprüche 1 bis 6, **dadurch gekennzeichnet, daß** sie zusätzlich eine radikalisch härtbare Verbindung und gegebenenfalls einen bei UV-Bestrahlung Radikale bildenden Initiator enthalten.

8. Verwendung der Reaktionsharzmischungen nach einem oder mehreren der Ansprüche 1 bis 7 zur Beschichtung oder Verklebung von nicht-transparenten Bauteilen, insbesondere von elektronischen Bauelementen und Baugruppen.

9. Verwendung der Reaktionsharzmischungen nach einem oder mehreren der Ansprüche 1 bis 7 zur Erzeugung von Strukturen.

10. Verwendung der Reaktionsharzmischungen nach einem oder mehreren der Ansprüche 1 bis 7 zur stereolithographischen Herstellung dreidimensionaler Gebilde.

## Claims

1. Curable reaction resin mixtures, especially for stereolithography, **characterized by** the following composition:
- a cationically curable monomer and/or oligomer (component A),
- an initiator of the following structure: 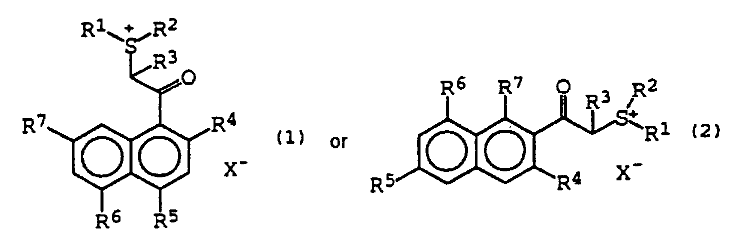 where
R¹ and R² are alkyl of 1 to 9 carbon atoms or cycloalkyl of 4 to 9 carbon atoms or combine to form a divalent aliphatic grouping of 4 to 7 carbon atoms,
R³ is H or alkyl of 1 to 9 carbon atoms,
R⁴, R⁵, R⁶ and R⁷ are H, alkyl of 1 to 9 carbon atoms or alkoxy of 1 to 9 carbon atoms, with the proviso that at least one of R⁴ to R⁷ is alkyl or alkoxy,
X⁻ is a non-nucleophilic anion, such as hexafluoroantimonate, hexafluoroarsenate and hexafluorophosphate, tetraphenylborate, tetra(perfluorophenyl)borate or trifluoromethanesulfonate,
- and optionally filler, pigment and/or additive.

2. Reaction resin mixtures according to Claim 1, **characterized in that** the initiator content is 0.01 to 10 wt%, based on component A.

3. Reaction resin mixtures according to Claim 1 or 2, **characterized in that** component A is an epoxy-functional compound, especially a compound having at least two epoxy groups per molecule.

4. Reaction resin mixtures according to Claim 3, **characterized in that** the epoxy-functional compound is an epoxidized terpene or α-alkene, a cycloaliphatic epoxide, an epoxy alcohol, a glycidyl ether or an epoxy-functionalized silicone.

5. Reaction resin mixtures according to Claim 1 or 2, **characterized in that** component A is a vinyl ether functional compound, especially a compound having at least two vinyl ether groups per molecule.

6. Reaction resin mixtures according to one or more of Claims 1 to 5, **characterized in that** they further comprise a polyfunctional hydroxyl compound.

7. Reaction resin mixtures according to one or more of Claims 1 to 6, **characterized in that** they further comprise a compound that cures by a free radical mechanism and optionally an initiator that forms free radicals under the effect of UV irradiation.

8. Use of the reaction resin mixtures according to one or more of Claims 1 to 7 for coating or adhering non-transparent components, especially electronic components and assemblies.

9. Use of the reaction resin mixtures according to one or more of Claims 1 to 7 for producing structures.

10. Use of the reaction resin mixtures according to one or more of Claims 1 to 7 for stereolithographically producing three-dimensional structures.

## Revendications

1. Mélanges durcissables de résines de réaction, notamment pour la stéréolithographie, **caractérisés par** la composition suivante :
- un monomère et/ou un oligomère pouvant durcir par voie cationique (constituant A),
- un initiateur ayant la structure suivante : 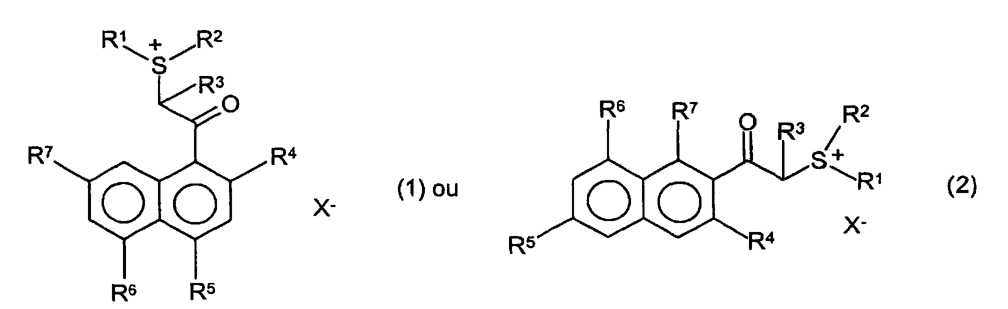 dans lesquels :
R¹ et R² sont alcoyles ayant de 1 à 9 atomes de carbone ou cycloalcoyles ayant de 4 à 9 atomes de carbone ou forment ensemble un groupement aliphatique divalent ayant de 4 à 7 atomes de carbone,
R³ est H ou alcoyle ayant de 1 à 9 atomes de carbone,
R⁴, R⁵, R⁶ et R⁷ sont H, alcoyles ayant de 1 à 9 atomes de carbone ou alcoxy ayant de 1 à 9 atomes de carbone, l'un au moins des radicaux R⁴ à R⁷ étant alcoyle ou alcoxy,
X⁻ est un anion non nucléophile comme de l'hexafluoroantimoniate, de l'hexafluoroarséniate et de l'hexafluorophosphate, du tétraphénylborate, du tétra(perfluorophényl)borate ou du trifluorométhanesulfonate,
- et le cas échéant une charge, un pigment et/ou un additif.

2. Mélanges de résines réactives suivant la revendication 1, **caractérisés en ce que** la teneur en initiateur est comprise entre 0,01 et 10 % en poids rapportée au constituant A.

3. Mélanges de résines réactives suivant la revendication 1 ou 2, **caractérisés en ce que** le constituant A a un composé à fonction époxy, notamment un composé ayant au moins deux groupes époxydes par molécule.

4. Mélanges de résines réactives suivant la revendication 3, **caractérisés en ce que** le composé à fonction époxy est un terpène époxydé ou un α-alcène époxydé, un époxyde cycloaliphatique, un époxyalcool, un oxyde glycidique ou une silicone à fonctionnalité époxy.

5. Mélanges de résines réactives suivant la revendication 1 ou 2, **caractérisés en ce que** le constituant A est un composé à fonction oxyde de vinyle, notamment un composé ayant au moins deux groupes oxyde de vinyle par molécule.

6. Mélanges de résines réactives suivant l'une ou plusieurs des revendications 1 à 5, **caractérisés en ce qu'**elles contiennent en outre un composé hydroxyle polyfonctionnel.

7. Mélanges de résines réactives suivant l'une ou plusieurs des revendications 1 à 6, **caractérisés en ce qu'**elles contiennent en outre un composé durcissable par voie radicalaire et le cas échéant un initiateur formant des radicaux par exposition à la lumière ultraviolette.

8. Utilisation des mélanges de résines réactives suivant l'une ou plusieurs revendications 1 à 7, pour revêtir ou pour coller des éléments non transparents, notamment des composants ou des modules électroniques.

9. Utilisation des mélanges de résines réactives suivant l'une ou plusieurs revendications 1 à 7 pour produire des structures.

10. Utilisation des mélanges de résines réactives suivant l'une ou plusieurs revendications 1 à 7 pour la production par stéréolithographie de structures en trois dimensions.
